(19) Europäisches Patentamt
European Patent Office
Office européen des brevets

(11) **EP 4 139 662 B1**

(12) **EUROPÄISCHE PATENTSCHRIFT**

(45) Veröffentlichungstag und Bekanntmachung des Hinweises auf die Patenterteilung:
**18.09.2024   Patentblatt 2024/38**

(21) Anmeldenummer: **21727094.1**

(22) Anmeldetag: **12.05.2021**

(51) Internationale Patentklassifikation (IPC):
**G01N 21/64** (2006.01)    **G01N 33/44** (2006.01)
**G01N 21/84** (2006.01)    **B07C 5/342** (2006.01)
**B29B 17/02** (2006.01)    **B07C 5/34** (2006.01)
**B29B 17/00** (2006.01)

(52) Gemeinsame Patentklassifikation (CPC):
**G01N 21/6408; B07C 5/3412; B29B 17/02; G01N 21/643; G01N 33/442;** B29B 2017/0021; B29B 2017/0203; B29B 2017/0279; B29B 2017/0282; G01N 2021/8411; G01N 2021/8416; Y02W 30/62

(86) Internationale Anmeldenummer:
**PCT/EP2021/062592**

(87) Internationale Veröffentlichungsnummer:
**WO 2021/228922 (18.11.2021 Gazette 2021/46)**

(54) **VERFAHREN UND SYSTEM ZUR HERSTELLUNG EINES KUNSTSTOFFMATERIALS**

METHOD AND SYSTEM FOR PRODUCING A PLASTIC MATERIAL

PROCÉDÉ ET SYSTÈME DE PRODUCTION D'UNE MATIÈRE PLASTIQUE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **15.05.2020   DE 102020113299**

(43) Veröffentlichungstag der Anmeldung:
**01.03.2023   Patentblatt 2023/09**

(73) Patentinhaber: **Sensor-Instruments Entwicklungs- und Vertriebs-GmbH 94169 Thurmansbang (DE)**

(72) Erfinder: **BRAUMANDL, Walter 94169 Thurmansbang (DE)**

(74) Vertreter: **Kramer Barske Schmidtchen Patentanwälte PartG mbB European Patent Attorneys Landsberger Strasse 300 80687 München (DE)**

(56) Entgegenhaltungen:
EP-A1- 1 801 148          WO-A1-2018/182437
JP-A- 2007 112 017       US-A- 5 397 819
US-A1- 2004 196 462   US-A1- 2018 088 103
US-A1- 2020 122 360

- **BRUNNER S ET AL: "Automated sorting of polymer flakes: Fluorescence labeling and development of a measurement system prototype", WASTE MANAGEMENT, vol. 38, 28 January 2015 (2015-01-28) - 28 January 2015 (2015-01-28), pages 49 - 60, XP029149914, ISSN: 0956-053X, DOI: 10.1016/J.WASMAN.2014.12.006**

**Beschreibung**

Die vorliegende Erfindung bezieht sich auf ein Verfahren und ein System zur Herstellung eines Kunststoffmaterials, insbesondere unter Verwendung eines recycelten Kunststoffrohmaterials. Darüber hinaus bezieht sich die Offenbarung auf Verfahren und Vorrichtungen zur Sortierung von Kunststoffobjekten, insbesondere im Rahmen eines Recyclingprozesses.

US 2018/0088103 A1 offenbart Fluorophore für eine Verifizierung von recyceltem Material.

US 2004/0196462 A1 offenbart ein Verfahren zum Einfärben eines Kunststoffs unter Verwendung von recyceltem Material.

US 2020/0122360 A1 offenbart ebenfalls ein Recycling-Verfahren.

BRUNNER S ET AL: "Automated sorting of polymer flakes: Fluorescence labeling and development of a measurement system prototype", WASTE MANAGEMENT, Bd. 38, Seiten 49-60, XP029149914, ISSN: 0956-053X, DOI: 10.1016/J.WASMAN.2014.12.006 betrifft eine automatische Sortierung von Polymer-Flakes.

Im Zuge eines Recyclingprozesses ist es häufig erforderlich, sicherzustellen, dass ein Produkt, das unter der Verwendung von Recyclinggut gefertigt wird, einen vorgegebenen Anteil des Recyclingguts enthält. Beispielsweise können gesetzliche Vorgaben bestehen, die den Anteil an Recyclinggut in einem neu hergestellten Produkt vorschreiben. Dies hat zur Folge, dass auch eine strenge Kontrolle der Menge des eingebrachten Recyclingguts notwendig ist.

Demzufolge ist es eine Aufgabe der vorliegenden Erfindung, ein Verfahren und ein System bereitzustellen, mit denen eine vorgegebene Menge an Recyclinggut in ein neu hergestelltes Kunststoffmaterial eingebracht werden kann, wobei nach der Herstellung eine Kontrolle des Anteils des eingebrachten Recyclingguts durchgeführt werden kann.

Diese Aufgabe wird gelöst mit den Merkmalen der Ansprüche 1 und 9. Weiterbildungen der Erfindung sind in den abhängigen Ansprüchen angegeben.

Bei der Wiederverwertung von Recyclingmaterial werden ein oder mehrere Marker (Kennzeichnungen, beispielsweise Partikel oder Stoffe mit bestimmten Eigenschaften) verwendet, um das Recyclingmaterial im Endprodukt detektieren zu können. Auf diese Weise kann kontrolliert werden, ob eine bestimmte Konzentration an Markern vorliegt, die einen Rückschluss auf die Menge bzw. den Anteil des recycelten Materials zulässt. Allerdings führt die Verwendung entsprechender Marker dazu, dass beispielsweise ein Teil des Recyclingmaterials bereits Marker enthält. Daher ist es nicht ausreichend, wenn dem Recyclingmaterial stets dieselbe Menge beispielsweise eines sogenannten Master-Batches mit einer vorgegebenen Konzentration der Marker zugegeben wird. Denn dies würde im Laufe der Zeit zu einer übermäßigen Erhöhung der Marker in dem zugegebenen Recyclingmaterial führen, was zum einen nicht erwünscht ist und zum anderen auch mit dem Vorwurf der Manipulation verbunden sein könnte. Denn zur Ermöglichung einer Kontrolle des Anteils an Recyclingmaterial ist es notwendig, dass das zugeführte Recyclingmaterial stets dieselbe Konzentration des Markers enthält.

Gemäß der vorliegenden Erfindung wird daher die Konzentration des Markers in dem Recyclingmaterial detektiert und in Abhängigkeit von der detektierten Konzentration die Menge des zugegebenen Master-Batches mit dem Marker geeignet eingestellt. Ferner kann die richtige Einstellung der Konzentration des Markers durch eine oder mehrere weitere Detektionseinheiten im Laufe des Herstellungsprozesses kontrolliert werden.

Ferner besteht beim Recyceln bzw. der Wiederverwertung von Kunststoffen eine der Aufgaben darin, die unterschiedlichen Kunststoffe sortenrein zu trennen. Dabei ist zu beachten, dass je nach Art der Wiederverwendung des Endprodukts nach Kunststoffart, Farbe und gegebenenfalls sogar nach dem Einsatzgebiet des Materials unterschieden werden muss. Beispielsweise sollte verhindert werden, dass das Material einer Kunststoffflasche, deren Inhalt aus Reinigungschemikalien bestand, zukünftig in der Matrix einer Kunststoffflasche für den Lebensmittelbereich wiederverwendet wird.

Diesbezüglich ist bekannt, zerkleinertes Kunststoffmaterial über ein Förderband zuzuführen und mittels eines Linienscanners in Form einer Infrarotkamera Spektren aufzuzeichnen, die anschließend ausgewertet werden. Der in einem Segment mittels spezifischem Infrarotspektrum erkannte Kunststoff wird dann im Anschluss entsprechend aussortiert.

Die oben genannten Infrarotkameras sind jedoch zum einen in der Anschaffung relativ kostenintensiv, und zum anderen können sie nicht an jedem Ort eingesetzt werden. Beispielsweise ist an Rücknahmestationen in Supermärkten, an denen das Recyclinggut als ganze Kunststoffobjekte einzeln zugeführt wird, eine Separation solcher einzelnen Objekte erforderlich.

Daher ist es ein weiteres Ziel der vorliegenden Offenbarung, ein Verfahren und eine Vorrichtung zur Sortierung von Kunststoffobjekten bereitzustellen, die auf möglichst praktikable und kostengünstige Weise einzelne Kunststoffobjekte nach ihrem Typ, beispielsweise dem verwendeten Kunststoffmaterial, der Farbe und gegebenenfalls sogar dem Verwendungszweck, sortieren kann.

Während bei den bekannten Sortiermaschinen Infrarotkameras verwendet werden, kann gemäß dem hierin beschriebenen Sortierverfahren ein sogenanntes Mehrbereichsverfahren im Infraroten verwendet werden. Dabei wird zur Erkennung der Kunststoffart der verwendete Wellenlängenbereich in mehrere Transmissionsbereiche (optische Fenster) unterteilt, und es wird die Transmission bzw. Reflexion in den jeweiligen optischen Fenstern detektiert. Auf diese Weise muss kein kompliziertes Spektrum aufgenommen und ausgewertet werden. Stattdessen können die detektieren Intensitäten mit mehreren Referenzgrößen (Vektoren) verglichen werden, und diejenige Referenzgröße mit der größten Übereinstimmung gibt dann die Art des Kunststoffmaterials an. Dies macht sich die Tatsache zu Nutze, dass unterschiedliche Kunststoffarten unterschiedliche charakteristische Transmissions- bzw. Reflexionsverhalten bei unterschiedlichen Wellenlängen, insbesondere im Infraroten, aufweisen.

Für den Fall, dass eine Sortierung auch nach der Farbe der Kunststoffe erfolgen soll, müssen vorzugsweise mehrere Detektorarten in ein System integriert werden. Zur Farberkennung werden dabei integrierte Weißlichtquellen (LED) in Verbindung mit den Infrarot- (IR-) Lichtquellen verwendet. Durch das Setzen geeigneter Flags kann das System dann entweder nur nach Farbe, nur nach Art des Kunststoffs oder sowohl nach Farbe als auch nach Art des Kunststoffs sortieren. Darüber hinaus wurde festgestellt, dass bei der Ermittlung der Kunststoffart anhand der detektierten Reflexionen im Infraroten eine genauere Detektion in Verbindung mit der Bestimmung der Farbe des Kunststoffs möglich ist, da sich diese in gewissem Maße auf die detektierten Intensitäten auswirken kann. Auch dies ist mit dem hierin offenbarten integrierten System problemlos möglich.

Ferner tritt beim Recycling von beispielsweise Kunststoffflaschen häufig das Problem auf, dass diese beispielsweise mit Papieretiketten versehen sind. Diese Papieretiketten können jedoch die Bestimmung der Kunststoffart anhand der reflektierten Intensitäten, beispielsweise im Infraroten, beeinflussen. Daher ist es notwendig, sicherzustellen, dass die Kunststoffart in einem Bereich des Kunststoffobjekts detektiert wird, in dem sich kein Papieretikett befindet. Diesbezüglich hat sich als vorteilhaft erwiesen, Ultraviolett- (UV-) Lichtquellen vorzusehen und das Kunststoffobjekt auch mit diesen zu beleuchten. Da Papier bei Beleuchtung mit UV-Licht eine Fluoreszenz im Sichtbaren, insbesondere im blauen Wellenlängenbereich, aufweist, kann anhand der Detektion bzw. dem Nichtvorhandensein der Fluoreszenz bestimmt werden, wann ein geeigneter Detektionsbereich zur Detektion der Kunststoffart vorliegt.

Zur Sortierung der Kunststoffobjekte nach der Erkennung der Art und/oder der Farbe derselben werden bevorzugt mehrere hintereinandergeschaltete Ausblasdüsen verwendet, die beispielsweise der Reihe nach Kunststoffobjekte aus bestimmtem Kunststoffmaterial aus dem Strom derselben aussondern. Entsprechende Ausblasdüsen führen ebenfalls die Separation nach der Farbe und gegebenenfalls dem Verwendungszweck durch.

Weitere Merkmale und Zweckmäßigkeiten ergeben sich aus der Beschreibung von Ausführungsbeispielen anhand der Figuren. Es zeigen:

    Fig. 1 eine schematische Ansicht einer Vorrichtung zur Sortierung von Kunststoffobjekten gemäß der vorliegenden Offenbarung;

    Fig. 2 eine schematische Ansicht einer Sende-/Empfangseinheit der Vorrichtung in Fig. 1;

    Fig. 3 eine Draufsicht auf eine beispielhafte Sende-/Empfangseinheit gemäß der vorliegenden Offenbarung;

    Fig. 4 eine teilweise Querschnittsansicht der Sende-/Empfangseinheit in Fig. 3;

    Fig. 5 einen vergrößerten Ausschnitt einer Weißlicht-Emissionseinheit der Sende-/Empfangseinheit in Fig. 3;

    Fig. 6 ein Diagramm zur Erläuterung unterschiedlicher Abklingverhalten von Lumineszenzen; und

    Fig. 7 eine schematische Ansicht eines Systems zum Herstellen eines Kunststoffmaterials gemäß der vorliegenden Erfindung.

Im Folgenden werden Ausführungsformen einer Vorrichtung zur Sortierung von Kunststoffobjekten und eines Systems zum Herstellen eines Kunststoffmaterials sowie zugehörige Verfahren unter Bezugnahme auf die Figuren beschrieben.

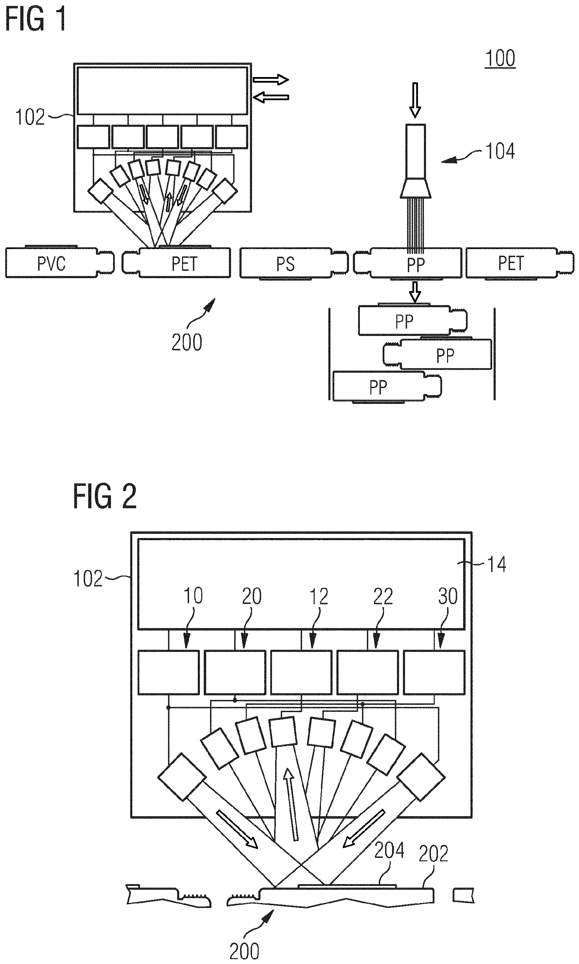

Fig. 1 zeigt eine schematische Ansicht einer Vorrichtung 100 zur Sortierung von Kunststoffobjekten 200, insbesondere im Rahmen eines Recyclingprozesses. Wie in Fig. 1 gezeigt, handelt es sich bei den Kunststoffobjekten beispielsweise um Kunststoffflaschen aus unterschiedlichen Kunststoffen, wie PVC, PET, PS, PP, etc. Die Kunststoffobjekte 200 werden über ein nicht gezeigtes Zufuhrsystem vereinzelt und einer geeigneten Förderanlage zugeführt, an der eine im Folgenden genauer beschriebene Sende-/Empfangseinheit 102 zur Detektion einer Art bzw. eines Typs des Kunststoffobjekts (des Kunststoffmaterials sowie gegebenenfalls einer Farbe und eines Verwendungszwecks desselben) vorgesehen ist. Auch wenn in Fig. 1 lediglich ein einzelner Strang einer entsprechenden Sortieranlage gezeigt ist, versteht sich, dass die vorliegende Erfindung auch bei Anlagen verwendet werden kann, bei denen das Recyclinggut zentral zugeführt und im Anschluss daran auf mehrere Stränge aufgeteilt wird. Es versteht sich ferner, dass in diesem Fall in jedem der Stränge eine Sende-/Empfangseinheit 102 enthalten ist.

Nach einer Detektion des Typs der einzelnen Kunststoffobjekte 200 werden diese durch eine herkömmliche Sortiereinheit 104, beispielsweise mehrere hintereinander angeordnete Ausblasdüsen, mit einer geeigneten Zeitsteuerung entsprechend aussortiert, beispielsweise in für jeden Typ speziell vorgesehene Behälter. Dies ist jedoch allgemein bekannt, so dass eine Beschreibung der Aussortierung hierin weggelassen wird.

Fig. 2 zeigt eine schematische Ansicht der Sende-/Empfangseinheit 102 in Fig. 1. Wie in Fig. 2 gezeigt, weist die Sende-/Empfangseinheit 102 eine IR-Sendeeinheit 10 zum Aussenden von IR-Licht (beispielsweise im Nahinfrarot- (NIR-) Bereich) in Richtung eines der Kunststoffobjekte 200 sowie eine IR-Detektionseinheit 12 zum Detektieren von IR-Intensitäten von an dem Kunststoffobjekt reflektiertem IR-Licht in mindestens zwei unterschiedlichen IR-Wellenlängenbereichen auf, was im Folgenden noch im Einzelnen erläutert wird. Darüber hinaus weist die Sende-/Empfangseinheit 102 eine Steuereinheit 14 zum Bestimmen eines Typs des Kunststoffobjekts durch Vergleichen mindestens der detektierten IR-Intensitäten mit einer Mehrzahl von Referenzgrößen, die jeweils unterschiedlichen Typen von Kunststoffobjekten zugeordnet sind, und Bestimmen der Referenzgröße mit der größten Übereinstimmung auf.

Die Sende-/Empfangseinheit 102 weist ferner eine Weißlicht-Emissionseinheit 20 und eine Farbdetektionseinheit 22 auf. Die Steuereinheit 14 ist dazu ausgebildet, die Weißlicht-Emissionseinheit 20 zum Emittieren von Weißlicht anzusteuern und basierend auf einer Detektion durch die Farbdetektionseinheit 22 eine Farbe des Kunststoffobjekts zu bestimmen. Ferner kann die Steuereinheit 14 dazu ausgebildet sein, den Typ des Kunststoffobjekts durch Vergleichen mindestens der detektierten IR-Intensitäten und von der Farbe des Kunststoffobjekts abgeleiteten Farbwerten mit den Mehrzahl von Referenzgrößen zu bestimmen.

Darüber hinaus weist die Sende-/Empfangseinheit 102 eine UV-Sendeeinheit 30 auf, wobei die Steuereinheit 14 dazu ausgebildet ist, die UV-Sendeeinheit 30 zum Aussenden von UV-Licht in der Richtung des Kunststoffobjekts anzusteuern und ansprechend darauf ein Fluoreszenzverhalten im sichtbaren Bereich zu bestimmen. Anhand des Fluoreszenzverhaltens bestimmt die Steuereinheit 14 einen IR-Detektionsbereich 202 an dem Kunststoffobjekt. Insbesondere ist der IR-Detektionsbereich 202 ein Bereich des Kunststoffobjekts, der frei von Etiketten, wie beispielsweise einem Papieretikett 204, ist.

Es versteht sich, dass für jede der Sende- bzw. Emissionseinheiten eine geeignete Treiberelektronik vorgesehen ist, über die die Steuereinheit 14 mit den jeweiligen Lichtquellen, beispielsweise eine oder mehrere LED, in Verbindung steht. Ebenso sind geeignete Empfängerelektroniken zur Detektion des reflektierten Lichts vorgesehen, wie dies in Fig. 2 für das reflektierte IR-Licht beispielhaft gezeigt ist. Die Steuereinheit ist auf bekannte Weise als ein Mikrocomputer oder Controller mit Elektronikkomponenten wie einem Speicher, einem Prozessor etc. ausgebildet und kann unter Verwendung geeigneter Software und Hardware die hierin beschriebenen Verfahren und Steuerungen sowie Detektionen und Berechnungen durchführen. Ferner kann die Steuereinheit mit einer externen Datenverarbeitungsvorrichtung in Verbindung stehen und zusammenwirken, um die Sende-/Empfangseinheit 102 und die Sortiereinheit 104 auf die hierin beschriebene Weise zu steuern. Bei einigen Ausführungsformen kann die Steuereinheit 14 separat von der Sende-/Empfangseinheit 102 vorgesehen sein und mit dieser in Verbindung stehen.

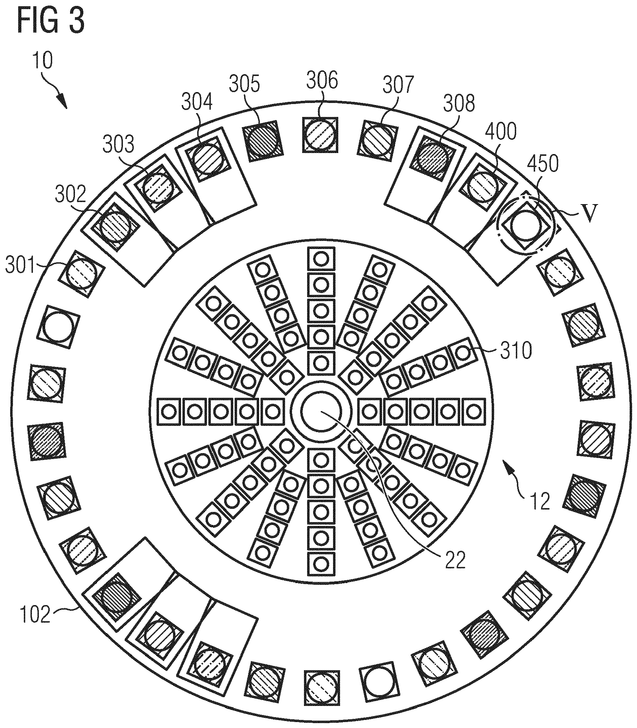

Die Steuereinheit 14 ist bei der vorliegenden Ausführungsform dazu ausgebildet, die IR-Sendeeinheit 10 zum zeitlich aufeinanderfolgenden Aussenden des IR-Lichts in den mindestens zwei unterschiedlichen IR-Wellenlängenbereichen anzusteuern. Dabei weist die IR-Sendeeinheit 10 mehrere unterschiedliche IR-LED 301-308 auf, beispielsweise drei oder mehr, insbesondere acht, wie bei dem in Fig. 3 gezeigten Beispiel. Die mehreren unterschiedlichen IR-LED 301-308 senden jeweils das Licht in den mehreren unterschiedlichen IR-Wellenlängenbereichen aus.

Wie in Fig. 3 gezeigt, können dabei für jeden IR-Wellenlängenbereich mehrere, beispielsweise drei, identische IR-LED 301-308 vorgesehen sein, die in einer Umfangsrichtung um ein Zentrum der IR-Sendeeinheit 10 in regelmäßigen Intervallen angeordnet sind. Jede der IR-LED 301-308 der vorliegenden Ausführungsform ist dazu ausgebildet, IR-Licht in einem Bereich zwischen 1000 nm und 2200 nm, insbesondere zwischen 1000 nm und 1700 nm, auszusenden. Beispielsweise können IR-LED 301-308 verwendet werden, die Zentralwellenlängen im Bereich von 1050 nm bis 1650 nm aufweisen, beispielsweise bei 1050 nm, 1100 nm, 1150 nm, 1250 nm, 1350 nm, 1450 nm, 1550 nm und 1650 nm.

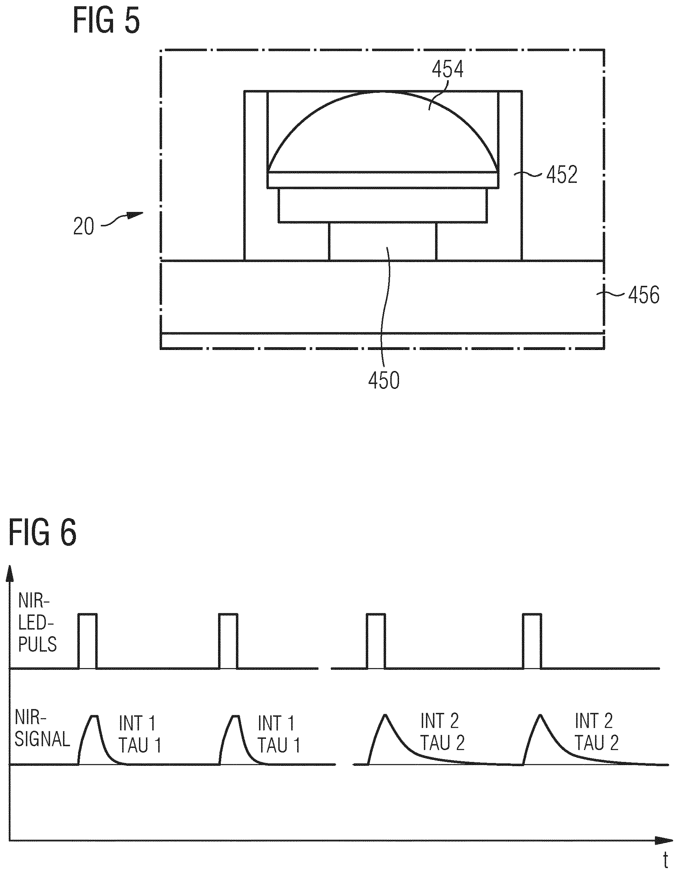

Wie in Fig. 3 gezeigt, sind auf demselben Radius wie die IR-LED 301-308 ferner eine Weißlicht-LED 450 und eine UV-LED 400 (mit einer Zentralwellenlänge beispielsweise von 365 nm) ebenfalls mit regelmäßigen Abständen insgesamt dreifach vorgesehen. Es versteht sich, dass bei anderen Ausführungsformen die Weißlicht-LED 450 und die UV-LED 400 bei unterschiedlichen Radien vorgesehen sein können. Fig. 5 zeigt eine vergrößerte Ansicht V der Weißlicht-LED 450. Wie in Fig. 5 gezeigt, ist die die Weißlicht-LED 450 in einem Gehäuse 452 aufgenommen, das von einer Linse 454 verschlossen ist. Die Weißlicht-LED 450 mit dem Gehäuse 452 ist auf einer Leiterplatte 456 angeordnet, beispielsweise einer Platine mit einem Aluminiumkern (nicht gezeigt), die zur effizienten Wärmeabfuhr dient. Es versteht sich, dass auch alle anderen LED der Sende-/Empfangseinheit 102 auf derartigen Leiterplatten vorgesehen sein können. Darüber hinaus ist für den Fachmann offensichtlich, dass einige oder alle der LED eine integrierte Linse (einen sogenannten Dom) anstelle der externen Linse 454 aufweisen können.

Wie ebenfalls in Fig. 3 gezeigt, weist die Detektionseinheit 12 bei der vorliegenden Ausführungsform mehrere parallel geschaltete breitbandige IR-Fotodioden 310 auf, die zum Detektieren des reflektierten IR-Lichts in den unterschiedlichen IR-Wellenlängenbereichen ausgebildet sind. Insbesondere sind die IR-LED 301-308 und die IR-Fotodioden 310 in der Sende-/Empfangseinheit 102 integriert, wobei die IR-Fotodioden 310 im Zentrum der IR-Sendeeinheit 10 angeordnet sind, beispielsweise als im Wesentlichen sternförmig angeordnete, in radialer Richtung verlaufende Reihen von IR-Fotodioden 310. Gegebenenfalls können die mehreren IR-Fotodioden 310 in mehrere, beispielsweise unterschiedliche Abstände zum Zentrum der IR-Sendeeinheit 10 aufweisende Gruppen von IR-Fotodioden unterteilt sein. Dies kann von Vorteil sein, da aufgrund der Parallelschaltung der IR-Fotodioden 310 die Kapazität zur Wiedergabe von schnellen Signalvorgängen unerwünscht groß werden kann, so dass die höherfrequenten Anteile der Signale möglicherweise nicht wiedergegeben werden können. Um dem vorzubeugen, kann deshalb die Aufteilung in die mehreren Gruppen (Zweige) hilfreich sein. Auf diese Weise wird die Kapazität jedes Zweigs verringert, und die Grenzfrequenz des Tiefpasses am Ausgang des jeweiligen Transimpedanzempfängers wird entsprechend erhöht. Eine Addition der einzelnen Zweige zu einem Gesamtsignal kann beispielsweise nach einer A/D-Wandlung mittels geeigneter Software in der Steuerung 14 erfolgen. Darüber hinaus kann durch die Unterteilung in mehrere Gruppen auch eine ortsabhängige (lokale) Auswertung erfolgen. Beispielsweise könnte bei anderen Ausführungsformen eine Anordnung der IR-Fotodioden 310 in mehreren, beispielsweise parallelen Reihen verwendet werden. Die jeweiligen LED werden dann entlang der Reihen bzw. zwischen denselben angeordnet. So können in der Längsrichtung der Reihen erneut mehrere Gruppen von IR-Fotodioden 310 festgelegt werden, beispielsweise jeweils die ersten drei jeder Reihe, dann die nächsten drei etc. (wobei drei lediglich beispielhaft ist und jede Gruppe aus mehr oder weniger IR-Fotodioden bestehen kann). Auch in diesem Fall werden die einzelnen IR-LED zeitlich versetzt zueinander angesteuert und die durch die jeweiligen Pulse ausgelösten Reflexionen in den unterschiedlichen Wellenlängenbereichen detektiert.

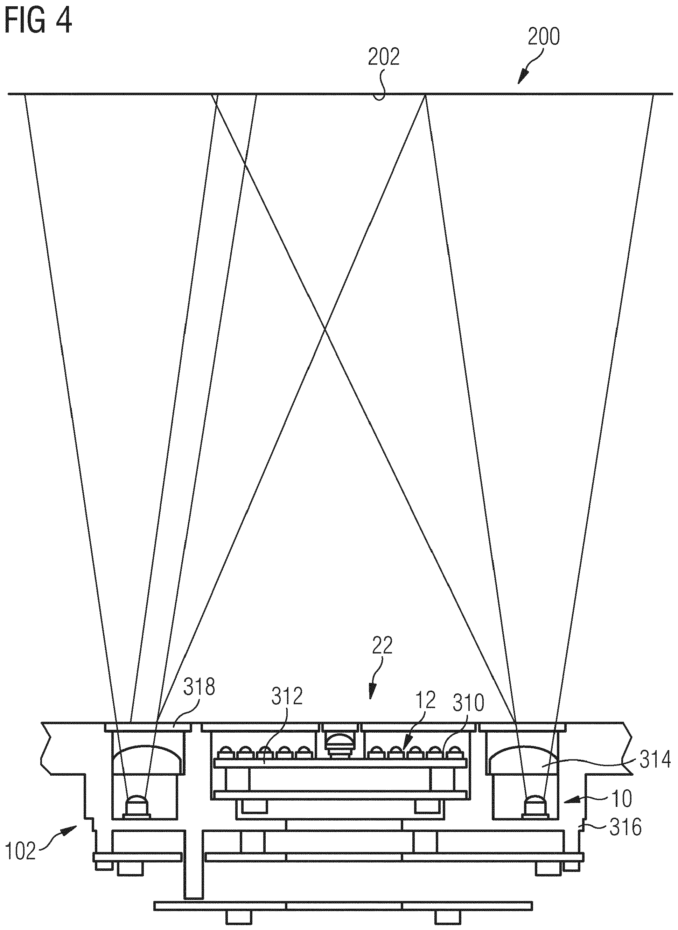

Fig. 4 zeigt eine Querschnittsansicht durch einen Teil der Sende-/Empfangseinheit 102 in Fig. 3. Wie in Fig. 4 gezeigt, ist die Farbdetektionseinheit 22 im Zentrum der Sende-/Empfangseinheit 102 angeordnet und dazu ausgebildet, das von der Weißlicht-LED 450 (siehe Fig. 3) ausgesandte und von den Kunststoffobjekten 200 reflektierte Licht zu detektieren. Das heißt, die Steuereinheit 14 ist dazu ausgebildet, die Weißlicht-Emissionseinheit 20 zum Emittieren von Weißlicht anzusteuern und basierend auf einer Detektion durch die Farbdetektionseinheit 22 eine Farbe des Kunststoffobjekts zu bestimmen. Die Einzelheiten einer solchen Farbbestimmung sind bekannt, so dass eine detaillierte Beschreibung weggelassen wird. Beispielsweise können die Intensitäten des reflektierten Lichts im roten, grünen und blauen Wellenlängenbereich detektiert werden und basierend darauf entsprechende Farbwerte (xyY oder L*a*b*) berechnet werden. Die Farbdetektionseinheit 22 dient ferner zur Detektion der Fluoreszenz aufgrund der Emission von UV-Licht durch die UV-Sendeeinheit 30. Es versteht sich, dass die UV-Sendeeinheit dazu zeitversetzt zur Weißlicht-Emissionseinheit 20 angesteuert wird.

Wie in Fig. 4 gezeigt, sind bei der beispielhaften Ausführungsform die IR-Sendeeinheit 10 und die IR-Detektionseinheit 12 (die IR-Fotodioden 310) auf unterschiedlichen Niveaus und bevorzugt in unterschiedlichen, voneinander getrennten Gehäuseabschnitten angeordnet und auf jeweiligen Leiterplatten 312, 316 montiert. Es versteht sich, dass vor jeder IR-LED 301-308 beispielsweise eine geeignete Optik mit einer Linse 314 und einem Filter 318 vorgesehen sein kann, um sicherzustellen, dass das ausgesandte IR-Licht auf geeignete Weise in Richtung einer durch das Zentrum der Sende-/Empfangseinheit 102 verlaufenden zentralen Achse ausgesandt und auf das Kunststoffobjekt 200, insbesondere den IR-Detektionsbereich 202 desselben, fokussiert wird.

Im Folgenden wird ein beispielhaftes Verfahren zur Sortierung der Kunststoffobjekte 200 unter Bezugnahme auf die Figuren beschrieben. Um den Typ bzw. die Eigenschaften oder Spezifikationen des Kunststoffobjekts, insbesondere die Art des verwendeten Kunststoffmaterials, die Farbe und den Verwendungszweck desselben, detektieren zu können, müssen zunächst geeignete Objektklassen definiert werden sowie den einzelnen Objektklassen zugeordnete Referenzgrößen bestimmt werden. Dies kann zum einen empirisch durchgeführt werden, beispielsweise während eines Betriebs der Anlage durch geeignete Lernroutinen (z.B. mittels KI, neuronalen Netzen oder anderer geeigneter Software), oder aber durch gezieltes Platzieren einzelner Objekte im korrekten Abstand und in der richtigen Position zu der Sensoroptik. Die einzelnen Objekte werden dann als die jeweiligen Referenzgrößen angelegt, die jeweils den Typ des Kunststoffobjekts angeben. Es versteht sich, dass die Referenzgrößen zusätzlich oder alternativ auch theoretisch ermittelt (berechnet) werden können, anhand des erwarteten Reflexionsverhaltens unterschiedlicher Materialien.

Beispielsweise enthält eine solche Referenzgröße Werte für jede der detektierten Intensitäten IR1-IRn (n = 2, 3, ...) im Infraroten, sowie einen Normierungsfaktor, der die Summe aus allen detektierten Intensitäten angibt. Bevorzugt werden dabei normierte Intensitäten ir1, ..., irn (n = 2, 3, ...) verwendet, wobei die normierten Intensitäten und der Normierungsfaktor B wie folgt gegeben sind:

$$\mathrm{ir}1 = \mathrm{IR}1/(\mathrm{IR}1 + \ldots + \mathrm{IR}n)$$

$$\mathrm{ir}n = \mathrm{IR}n/(\mathrm{IR}1 + \ldots + \mathrm{IR}n)$$

$$B = \mathrm{IR}1 + \ldots + \mathrm{IR}n)$$

Weitere Bestandteile der jeweiligen Referenzgröße sind beispielsweise drei Farbwerte (z.B. x, y, INT), die unter Verwendung der Farbdetektionseinheit 22 erhalten werden, sowie mehrere Werte (z.B. UV-x, UV-y, UV-INT), die einem aufgrund von Emission im Ultravioletten detektierten Licht, beispielsweise im sichtbaren Bereich (in diesem Fall kann zur Detektion die Farbdetektionseinheit 22 verwendet werden), zugeordnet sind. Das heißt, bei dem vorliegenden Beispiel enthält jede Referenzgröße 3 + 3 + 9 Werte. Bei einigen Ausführungsformen müssen nicht alle Werte ir1, ..., irn berechnet bzw. verwendet werden, da natürlich gilt im = 1 - (ir1 + ... + irn-1). Ferner können bei einigen Ausführungsformen beliebige Kombinationen aus den Werten als die Referenzgröße bzw. für die Bestimmung der Referenzgröße mit der größten Übereinstimmung verwendet werden.

Während des Betriebs der Sortieranlage erfolgt zunächst eine Detektion bzw. Triggerung eines Objekts. Dies erfolgt dabei beispielsweise mittels der Farbdetektionseinheit 22, die beispielsweise eine von einem Referenzwert abweichende Farbe detektiert. Im Anschluss werden die weiteren für den Vergleich mit den Referenzgrößen benötigten Werte detektiert. Das heißt, beispielsweise werden die LED 301-308 von der Steuereinheit 14 der Reihe nach angesteuert, um IR-Licht auszusenden, und das reflektierte IR-Licht in den jeweiligen Wellenlängenbereich wird durch die IR-Detektionseinheit 12 detektiert, die synchron oder leicht phasenverschoben zu der Emission der IR-Pulse angesteuert wird. Sofern nicht bereits durch den Trigger-Vorgang erhalten, werden im Anschluss daran die Farbwerte durch Aktivieren der Weißlicht-LED 450 und Detektieren des reflektierten Lichts mittels der Farbdetektionseinheit 22 erhalten. Optional kann auch die UV-LED 400 aktiviert werden, um Farbwerte des aufgrund des UV-Lichts ausgesandten reflektierten oder aufgrund von Lumineszenz emittierten Lichts im sichtbaren Bereich zu detektieren.

Es versteht sich, dass die einzelnen Detektionsvorgänge während des Passierens eines Kunststoffobjekts 200 binnen kürzester Zeit mehrmals durchgeführt werden können. Auf diese Weise kann, wie oben bereits erläutert, durch die UV-Sendeeinheit 30 sichergestellt werden, dass ein Detektionsbereich 202 für die Detektion verwendet wird, in dem kein Papieretikett oder dergleichen an dem Kunststoffobjekt ist. Für den Fall, dass mehrere Detektionen an einem Kunststoffobjekt durchgeführt werden, können auf geeignete Weise Mittelwerte gebildet werden oder aus der Reihe fallende Messwerte unberücksichtigt bleiben.

Zur Bestimmung des Typs des Kunststoffobjekts 200 wird nun beispielsweise im Rahmen eines "Best Hit"-Verfahrens diejenige mehrdimensionale Referenzgröße ermittelt, die die beste Übereinstimmung mit den detektierten Werten aufweist. Für den Fachmann ist dabei offensichtlich, wie in dem m-dimensionalen Vektorraum (m = 2, 3, ...), der durch die Gesamtheit der einzelnen Detektionswerte aufgespannt wird, der minimale Abstand zu einer der Referenzgrößen bestimmt werden kann. Die Referenzgröße mit dem minimalen Abstand ist dann diejenige Referenzgröße, die den Typ des Kunststoffobjekts 200 angibt. Die Referenzgröße kann ferner angeben, ob eine Sortierung nach dem verwendeten Kunststoffmaterial (nach der Art) und/oder nach der Farbe erfolgen soll. Das heißt, geeignete Flags können entsprechend gesetzt sein.

Nachdem die Referenzgröße mit der größten Übereinstimmung gefunden worden ist, kann die Steuereinheit 14 die Sortiereinheit 104 (bzw. die mehreren Ausblasdüsen derselben) auf geeignete Weise zeitsynchron ansteuern, um das Kunststoffobjekt 200 je nach seinem Typ dem geeigneten Sortierbehälter zuzuführen. Wie bereits erwähnt, ist es dabei von Vorteil, auch die jeweiligen Farbwerte zum Abgleichen mit den Referenzgrößen zu verwenden, auch wenn lediglich nach der Kunststoffart sortiert werden soll.

Ferner kann die Genauigkeit, wie ebenfalls bereits erwähnt, dadurch erhöht werden, dass ein geeigneter Detektionsbereich 202 bestimmt wird, beispielsweise als ein Bereich, in dem eine Intensität einer Fluoreszenz im sichtbaren Bereich, beispielsweise im blauen Wellenlängenbereich, bei Bestrahlung mit UV-Licht unterhalb eines vorbestimmten Schwellenwerts liegt.

Wie bereits erläutert, kann es zusätzlich auch wünschenswert sein, das Kunststoffobjekt nach seinem Verwendungszweck unterschiedlich einzusortieren. Dies kann beispielsweise durch Detektieren des Vorhandenseins oder Fehlens eines oder mehrerer Marker, beispielsweise Stoffe, die eine Lumineszenz im sichtbaren Bereich oder im IR-Bereich aufweisen, ermöglicht werden. So können beispielsweise Behälter bzw. Verpackungen von Lebensmitteln, Behälter für Reinigungsmittel wie Flüssigseifen, Behälter zur Aufbewahrung von Abflussreinigern, etc. geeignet sortiert werden. Es können beispielsweise phosphoreszierende Down-Converter eingesetzt werden, sowie Marker, die eine Anregung im UV- Bereich aufweisen und eine Sekundäremission in sichtbaren Wellenlängenbereichen aufweisen. Natürlich können auch Marker verwendet werden, die im Sichtbaren angeregt werden und im roten Wellenlängenbereich oder im Nahinfrarotbereich nachleuchten. Schließlich können auch Marker verwendet werden, die mit IR-Licht angeregt werden und bei niedrigeren Energien emittieren. Insbesondere können jeweilige Zeitkonstanten sowie Anfangsintensitäten der Lumineszenz bzw. des Nachleuchtens erfasst werden. Dies ist beispielsweise in Fig. 6 gezeigt. Dort sind zwei unterschiedliche Marker gezeigt, die zwar im Wesentlichen dieselbe Anfangsintensität des Nachleuchtens aufweisen, jedoch unterschiedliche Abklingzeiten $\tau1$ bzw. $\tau2$ haben. Das heißt, bei dem in Fig. 6 gezeigten Beispiel könnte so zwischen zwei Kunststoffen, die zwar aus identischem Material bestehen und eine identische Farbe aufweisen, jedoch für unterschiedliche Zwecke verwendet werden sollen, unterschieden werden. Eine Sortierung von Kunststoffobjekten nach ihrem Verwendungszweck wird beispielsweise nach der oben beschriebenen Bestimmung des Typs der Kunststoffobjekte und einer entsprechenden Vorsortierung durchgeführt.

Es versteht sich, dass die oben im Einzelnen beschriebene Sende-/Empfangseinheit 102 lediglich beispielhaft ist, und selbstverständlich mehr oder weniger als acht unterschiedliche IR-LED eingesetzt werden können. Ferner kann die Anordnung der IR-LED von der in Fig. 3 gezeigten Anordnung abweichen. Entsprechendes gilt für die Weißlicht-LED 450 und die UV-LED 400. Ferner können auch die Anzahl, Anordnung und Position der einzelnen LED bezüglich der IR-Fotodioden 310 unterschiedlich sein. Entsprechendes gilt auch für die Farbdetektionseinheit 22. Darüber hinaus ist es nicht zwingend notwendig, als IR-Lichtquelle IR-LED zu verwenden, die gepulst betrieben werden. Beispielsweise ist es auch denkbar, eine kontinuierliche IR-Lichtquelle einzusetzen und die Reflexion durch für die jeweiligen Wellenlängenbereiche vorgesehene IR-Fotodioden (gegebenenfalls unter Einsatz geeigneter Filter) zu detektieren.

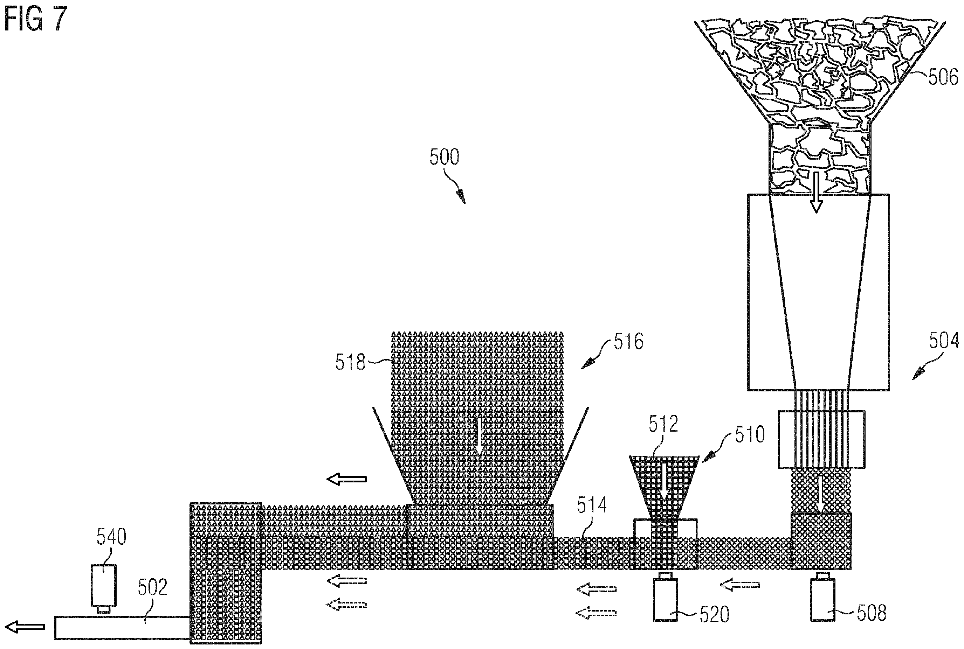

Die oben beschriebenen Marker, die beispielsweise zur Identifizierung von Kunststoffobjekten, die in unterschiedlichen Bereichen einzusetzen sind, verwendet werden, können ebenfalls dazu dienen, festzustellen, welcher prozentuale Anteil an Recyclingmaterial in einem Endprodukt enthalten ist. Nach der Beimengung des Markers sollte das Recyclinggut eine konstant bleibende Konzentration von Markern aufweisen. Es versteht sich, dass dabei eine Kontrolle erfolgen muss, damit auf Seite des Herstellers nicht übermäßig Material mit dem Marker zugegeben wird, um die Konzentration zu erhöhen, so dass nicht die erforderliche Menge an Recyclinggut verwendet werden muss. Ein System bzw. ein Verfahren, mit dem eine konstante (vorgegebene) Markerkonzentration erreicht werden kann, wird im Folgenden unter Bezugnahme auf Fig. 7 beschrieben.

Wie in Fig. 7 gezeigt, weist ein System 500 zum Herstellen eines Kunststoffmaterials 502, das zum Teil aus wiederverwertetem (recyceltem) Kunststoff besteht, eine erste Zufuhreinheit 504 zum Zuführen eines ersten Kunststoffrohmaterials 506, das insbesondere eins recyceltes Kunststoffrohmaterial ist, auf. Ferner weist das System 500 eine erste Detektionseinheit 508 zum Bestimmen einer Konzentration eines Markers, der eine Lumineszenz aufweist, in dem zugeführten ersten Kunststoffrohmaterial durch Detektion einer Intensität der Lumineszenz auf. Dabei kann, wie in Bezug auf Fig. 6 beschrieben, beispielsweise die Konzentration des Markers anhand einer Anfangsintensität der Lumineszenz des die erste Detektionseinheit 508 passierenden Materials bestimmt werden. Bei diesem Material handelt es sich beispielsweise um zerkleinertes und bereits nach Kunststoffart vorsortiertes Recyclingmaterial, das einem Extruder zugeführt, aufgeschmolzen, in Stränge verarbeitet und anschließend zu Granulat zerkleinert wurde. Insbesondere kann die Detektionseinheit 508 dazu ausgebildet sein, nach einem bestimmten Marker zu suchen, der eine bekannte Abklingzeit aufweist. Ferner kann anhand der Anfangsintensität die Konzentration des Markers gemessen werden. Das heißt, anhand des Absolutwerts der Anfangsintensität kann rechnerisch die Konzentration des Markers erhalten werden. Falls notwendig, kann eine geeignete Kalibrierung der Detektionseinheit 508 erfolgen, so dass bei einer Detektion in einem vorgegebenen Abstand eine eindeutige Zuordnung zwischen Anfangsintensität und Markerkonzentration erfolgen kann.

Für den Fall, dass in ein und derselben Anlage unterschiedliche Kunststoffe verarbeitet werden, versteht sich, dass unterschiedliche Marker detektiert werden können. Gegebenenfalls sind dazu mehrere Detektionseinheiten 508 vorgesehen. Für den Fall, dass die Art des Kunststoffmaterials nicht bekannt ist, sondern im Rahmen des Prozesses zuerst bestimmt werden muss, kann auf diese Weise nach unterschiedlichen Markern gesucht werden, eine Anfangsintensität und/oder eine Abklingkonstante von Lumineszenzen zur Identifizierung eines Markers bestimmt werden, und basierend darauf die weitere Verarbeitung erfolgen, was im Folgenden noch erläutert wird.

Das System 500 weist ferner eine zweite Zufuhreinheit 510 zum Zuführen eines zweiten Kunststoffrohmaterials 512, das eine höhere Konzentration des Markes als das erste Kunststoffrohmaterial 506 aufweist, zu dem ersten Kunststoffrohmaterial in Abhängigkeit von der bestimmten Konzentration des Markes in dem ersten Kunststoffrohmaterial auf. Insbesondere führt die zweite Zufuhreinheit 510 ein sogenanntes Master-Batch mit einer vergleichsweise hohen (festgelegten bzw. bekannten) Konzentration des Markers zu. Dies geschieht auf eine Weise, so dass eine resultierende Mischung 514 aus dem ersten und dem zweiten Kunststoffrohmaterial erhalten wird, die eine vorgegebene Soll-Konzentration des Markers aufweist. Die Soll-Konzentration bestimmt sich dabei nach dem Anteil, den das Recyclingmaterial (die Mischung 514) später in dem Endprodukt haben soll. Dazu kann die Soll-Konzentration eine vorgegebene Konzentration sein, so dass basierend darauf dann anhand einer Detektion an dem Endprodukt ein Rückschluss auf den Anteil des Recyclingmaterials gezogen werden kann. Es versteht sich, dass die Menge an zweitem Kunststoffrohmaterial (Master-Batch) im Vergleich zu dem ersten Kunststoffrohmaterial gering bzw. vernachlässigbar ist, beispielsweise etwa 1 bis 2 Prozent beträgt. Ferner versteht sich, dass für den Fall, dass das erste Kunststoffrohmaterial bereits die Soll-Konzentration aufweist, keine Zufuhr des zweiten Kunststoffrohmaterials erfolgt.

Die Mischung 514 wird einer dritten Zufuhreinheit 516 zugeführt, die ein drittes Kunststoffrohmaterial (beispielsweise Neumaterial) 518, das den Marker nicht enthält, in einem vorbestimmten Verhältnis zu der Mischung 514 zuführt. Auf diese Weise wird das Kunststoffmaterial 502 erhalten, das einen vorbestimmten Anteil im Wesentlichen des ersten Kunststoffrohmaterials 506 (d.h. der Mischung 514) enthält.

Gegebenenfalls kann eine zweite Detektionseinheit 520 zum Bestimmen einer Konzentration des Markers in dem zweiten Kunststoffrohmaterial 512 vor dem Zuführen desselben und/oder in der Mischung 514 vorgesehen sein. Die zweite Zufuhreinheit 510 ist ggf. zum (weiteren) Einstellen bzw. Regeln der Menge des zugeführten zweiten Kunststoffrohmaterials 512 basierend auf der bestimmten Konzentration des Markers in dem zweiten Kunststoffrohmaterial und/oder der Mischung 514, so dass die Mischung die Soll-Konzentration des Markers aufweist, ausgebildet. Es versteht sich, dass die jeweiligen Zufuhreinheiten jeweilige Steuereinheiten aufweisen oder mit solchen verbunden sind, die die Komponenten wie Düsen, etc. der jeweiligen Zufuhreinheiten geeignet steuern können.

Bei einigen Ausführungsformen kann das zweite Kunststoffmaterial eine im Voraus festgelegte Konzentration des Markers aufweisen. Das heißt, das zugeführte Master-Batch kann eine bekannte Konzentration des Markers aufweisen. Diese Konzentration kann jedoch durch die zweite Detektionseinheit 520 während des Prozesses kontinuierlich überprüft werden, um gegebenenfalls Abweichungen korrigieren zu können. Ferner können die Detektionsergebnisse der zweiten Detektionseinheit 520 zur Prozessüberwachung und Dokumentation gespeichert werden. Beispielsweise kann zur Gewährleistung der Einhaltung der Vorschriften im Hinblick auf den Anteil des recycelten Materials bzw. die Markerkonzentration ein sicheres, ggf. verschlüsseltes und vor Manipulation geschütztes Verfahren verwendet werden. Alternativ kann auch die Steuersoftware, die beispielsweise die zweite Zufuhreinheit 510 in Abhängigkeit von der Detektion durch die Detektionseinheiten 508 und 520 steuert, so ausgebildet sein, dass Manipulationen ausgeschlossen sind.

Weiter stromabwärts wird das Rohmaterial ohne Marker (in der Regel Neumaterial) der Mischung 514 beigemengt, in dem beispielsweise vom Gesetzgeber vorgegebenen Verhältnis. Abschließend kann dann nach Einschmelzen und Extrudieren des erhaltenen Kunststoffmaterials 502 eine weitere Überprüfung mittels einer dritten Detektionseinheit 540 erfolgen. Auf diese Weise kann nochmals überprüft werden, ob die gewünschte Konzentration des Markers vorliegt.

Mit dem oben beschriebenen System wird insbesondere der Tatsache Rechnung getragen, dass das recycelte Kunststoffmaterial 506 bereits unterschiedliche Konzentrationen von Markern enthalten kann. Das heißt, es wird nur so viel des Master-Batches 512 zugeführt, wie benötigt wird, um die notwendige Soll-Konzentration des Markers in der Mischung 514 und damit im Endprodukt 502 zu ergeben. Somit kann eine unnötige Zugabe des Markers vermieden werden.

Es wird explizit festgehalten, dass alle Bereichsangaben oder Angaben von Gruppen von Einheiten jeden möglichen Zwischenwert oder Untergruppe von Einheiten zum Zweck der ursprünglichen Offenbarung ebenso wie zum Zweck des Einschränkens der beanspruchten Erfindung offenbaren, insbesondere auch als Grenze einer Bereichsangabe.

**Patentansprüche**

1. Verfahren zur Herstellung eines Kunststoffmaterials (502), mit folgenden Schritten:

   Zuführen eines ersten Kunststoffrohmaterials (506);
   Bestimmen einer Konzentration eines Markers, der eine Lumineszenz aufweist, in dem zugeführten ersten Kunststoffrohmaterial durch Detektion einer Intensität der Lumineszenz;
   Zuführen eines zweiten Kunststoffrohmaterials (512), das eine höhere Konzentration des Markers als das erste Kunststoffrohmaterial aufweist, zu dem ersten Kunststoffrohmaterial (506), wenn die Konzentration des Markers eine Soll-Konzentration unterschreitet, in Abhängigkeit von der bestimmten Konzentration des Markers in dem ersten Kunststoffrohmaterial, so dass eine Mischung (514) aus dem ersten und dem zweiten Kunststoffrohmaterial erhalten wird, die eine Soll-Konzentration des Markers aufweist; und
   Zuführen eines dritten Kunststoffrohmaterials (518), das den Marker nicht enthält, zu der Mischung (514) in einem vorbestimmten Verhältnis zur Mischung, so dass das Kunststoffmaterial (502) erhalten wird, das einen vorbestimmten Anteil der Mischung (514) enthält.

2. Verfahren nach Anspruch 1, ferner mit

   Bestimmen einer Konzentration des Markers in dem zweiten Kunststoffrohmaterial (512) vor dem Zuführen desselben und/oder
   Bestimmen einer Konzentration des Markers in der Mischung (514)
   und
   Regeln der Menge des zugeführten zweiten Kunststoffrohmaterials (512) basierend auf der bestimmten Konzentration des Markers in dem zweiten Kunststoffrohmaterial und/oder der Mischung, so dass die Mischung (514) die Soll-Konzentration des Markers aufweist.

3. Verfahren nach Anspruch 1 oder 2, bei dem das zweite Kunststoffrohmaterial (512) eine im Voraus festgelegte Konzentration des Markers aufweist.

4. Verfahren nach einem der Ansprüche 1 bis 3, ferner mit

   Bestimmen der Konzentration des Markers in dem Kunststoffmaterial (502) nach dem Zuführen des dritten Kunststoffrohmaterials (518) und Vergleichen der bestimmten Konzentration mit einer basierend auf dem vorbestimmten Anteil und der Soll-Konzentration berechneten Konzentration.

5. Verfahren nach einem der Ansprüche 1 bis 4, ferner mit
Zerkleinern des ersten Kunststoffrohmaterials (506) vor dem Zuführen desselben.

6. Verfahren nach einem der Ansprüche 1 bis 5, bei dem die Konzentration des Markers anhand einer Anfangsintensität der Lumineszenz des einen Sensor passierenden Materialstroms bestimmt wird.

7. Verfahren nach einem der Ansprüche 1 bis 6, bei dem die Lumineszenz im Infraroten detektiert wird.

8. Verfahren nach einem der Ansprüche 1 bis 7, ferner mit

Bestimmen der Anfangsintensität und/oder einer Abklingkonstante der Lumineszenz zur Identifizierung des Markers und
Auswählen des zweiten Kunststoffrohmaterials (512) und/oder des dritten Kunststoffrohmaterials (518) aus mehreren Materialien in Abhängigkeit von der Identifizierung.

9. System (500) zum Herstellen eines Kunststoffmaterials (502), mit:

einer ersten Zufuhreinheit (504) zum Zuführen eines ersten Kunststoffrohmaterials (506);
einer ersten Detektionseinheit (508) zum Bestimmen einer Konzentration eines Markers, der eine Lumineszenz aufweist, in dem zugeführten ersten Kunststoffrohmaterial durch Detektion einer Intensität der Lumineszenz;
einer zweiten Zufuhreinheit (510) zum Zuführen eines zweiten Kunststoffrohmaterials (512), das eine höhere Konzentration des Markers als das erste Kunststoffrohmaterial (506) aufweist, zu dem ersten Kunststoffrohmaterial, wenn die Konzentration des Markers eine Soll-Konzentration unterschreitet, in Abhängigkeit von der bestimmten Konzentration des Markers in dem ersten Kunststoffrohmaterial, so dass eine Mischung (514) aus dem ersten und dem zweiten Kunststoffrohmaterial erhalten wird, die eine Soll-Konzentration des Markers aufweist; und
einer dritten Zufuhreinheit (516) zum Zuführen eines dritten Kunststoffrohmaterials (518), das den Marker nicht enthält, zu der Mischung (514) in einem vorbestimmten Verhältnis zur Mischung, so dass das Kunststoffmaterial (502) erhalten wird, das einen vorbestimmten Anteil der Mischung (514) enthält.

10. System nach Anspruch 9, ferner mit

einer zweiten Detektionseinheit (520) zum Bestimmen einer Konzentration des Markers in dem zweiten Kunststoffrohmaterial (512) vor dem Zuführen desselben und/oder in der Mischung (514),
bei dem die zweite Zufuhreinheit (510) zum Einstellen der Menge des zugeführten zweiten Kunststoffrohmaterials (512) basierend auf der bestimmten Konzentration des Markers in dem zweiten Kunststoffrohmaterial und/oder der Mischung (514), so dass die Mischung die Soll-Konzentration des Markers aufweist, ausgebildet ist.

**Claims**

1. A method for manufacturing a plastic material (502), the method comprising:

supplying a first plastic raw material (506);
determining a concentration of a marker having a luminescence in the supplied first plastic raw material by detecting an intensity of the luminescence;
supplying a second plastic raw material (512) having a higher concentration of the marker than the first plastic raw material to the first plastic raw material (506) in case the concentration of the marker is below a target concentration, depending on the determined concentration of the marker in the first plastic raw material, such that a mixture (514) of the first and second plastic raw materials is obtained, which has a target concentration of the marker; and
supplying a third plastic raw material (518) that does not contain the marker to the mixture (514) in a predetermined ratio to the mixture, such that the plastic material (502) containing a predetermined portion of the mixture (514) is obtained.

2. The method of claim 1, further comprising

determining a concentration of the marker in the second plastic raw material (512) prior to supplying the same and/or
determining a concentration of the marker in the mixture (514),
and
controlling the amount of the supplied second plastic raw material (512) based on the determined concentration of the marker in the second plastic raw material and/or the mixture, such that the mixture (514) has the target concentration of the marker.

3. The method of claim 1 or 2, wherein the second plastic raw material (512) has a concentration of the marker that is fixed in advance.

4. The method of one of claims 1 to 3, further comprising

   determining the concentration of the marker in the plastic material (502) after supplying the third plastic raw material (518) and
   comparing the determined concentration to a concentration calculated based on the predetermined portion and the target concentration.

5. The method of one of claims 1 to 4, further comprising
   shredding the first plastic raw material (506) prior to supplying the same.

6. The method of one of claims 1 to 5, wherein the concentration of the marker is determined from an initial intensity of the luminescence of the material flow passing a sensor.

7. The method of one of claims 1 to 6, wherein the luminescence is detected in the infrared.

8. The method of one of claims 1 to 7, further comprising

   determining the initial intensity and/or a decay constant of the luminescence for identification of the marker and
   selecting the second plastic raw material (512) and/or the third plastic raw material (518) from a plurality of materials depending on the identification.

9. A system (500) for manufacturing a plastic material (502), comprising:

   a first supply unit (504) for supplying a first plastic raw material (506);
   a first detection unit (508) for determining a concentration of a marker having a luminescence in the supplied first plastic raw material by detecting an intensity of the luminescence;
   a second supply unit (510) for supplying a second plastic raw material (512) having a higher concentration of the marker than the first plastic raw material (506) to the first plastic raw material in case the concentration of the marker is below a target concentration, depending on the determined concentration of the marker in the first plastic raw material, such that a mixture (514) of the first and second plastic raw materials is obtained, which has a target concentration of the marker; and
   a third supply unit (516) for supplying a third plastic raw material (518) that does not contain the marker to the mixture (514) in a predetermined ratio to the mixture, such that the plastic material (502) containing a predetermined portion of the mixture (514) is obtained.

10. The system of claim 9, further comprising

    a second detection unit (520) for determining a concentration of the marker in the second plastic raw material (512) prior to supplying the same and/or in the mixture (514),
    wherein the second supply unit (510) is configured to set the amount of the supplied second plastic raw material (512) based on the determined concentration of the marker in the second plastic raw material and/or the mixture (514), such that the mixture has the target concentration of the marker.

**Revendications**

1. Procédé de fabrication d'un matériau plastique (502), comprenant les étapes suivantes :

   fournir une première matière brute plastique (506) ;
   déterminer une concentration d'un marqueur présentant une luminescence dans la première matière brute plastique fournie en détectant une intensité de la luminescence ;
   fournir une deuxième matière brute plastique (512), qui présente une concentration plus élevée du marqueur que la première matière brute plastique, à la première matière brute plastique (506), lorsque la concentration du marqueur est inférieure à une concentration cible, en fonction de la concentration déterminée du marqueur dans la première matière brute plastique, de sorte qu'un mélange (514) de la première et de la deuxième matière brute plastique soit obtenu, présentant une concentration cible du marqueur ; et
   fournir une troisième matière brute plastique (518), qui ne contient pas le marqueur, au mélange (514) dans un rapport prédéterminé au mélange,
   de sorte que le matériau plastique (502) soit obtenu, contenant une proportion prédéterminée du mélange (514).

2. Procédé selon la revendication 1, comprenant en outre :

   déterminer une concentration du marqueur dans la deuxième matière brute plastique (512) avant de la fournir et/ou
   déterminer une concentration du marqueur dans le mélange (514) et

réguler la quantité de la deuxième matière brute plastique fournie (512) en fonction de la concentration déterminée du marqueur dans la deuxième matière brute plastique et/ou le mélange, de sorte que le mélange (514) présente la concentration cible du marqueur.

3. Procédé selon la revendication 1 ou 2, dans lequel la deuxième matière brute plastique (512) présente une concentration prédéterminée du marqueur.

4. Procédé selon l'une des revendications 1 à 3, comprenant en outre :

déterminer la concentration du marqueur dans le matériau plastique (502) après avoir fourni la troisième matière brute plastique (518) et comparer la concentration déterminée avec une concentration calculée en fonction de la proportion prédéterminée et de la concentration cible.

5. Procédé selon l'une des revendications 1 à 4, comprenant en outre :
broyer la première matière brute plastique (506) avant de la fournir.

6. Procédé selon l'une des revendications 1 à 5, dans lequel la concentration du marqueur est déterminée en fonction d'une intensité initiale de la luminescence du flux de matériau passant par un capteur.

7. Procédé selon l'une des revendications 1 à 6, dans lequel la luminescence est détectée dans l'infrarouge.

8. Procédé selon l'une des revendications 1 à 7, comprenant en outre :

déterminer l'intensité initiale et/ou une constante de décroissance de la luminescence pour identifier le marqueur et

sélectionner la deuxième matière brute plastique (512) et/ou la troisième matière brute plastique (518) parmi plusieurs matériaux en fonction de l'identification.

9. Système (500) pour la fabrication d'un matériau plastique (502), comprenant :

une première unité d'alimentation (504) pour fournir une première matière brute plastique (506) ;
une première unité de détection (508) pour déterminer une concentration d'un marqueur présentant une luminescence dans la première matière brute plastique fournie en détectant une intensité de la luminescence ;

une deuxième unité d'alimentation (510) pour fournir une deuxième matière brute plastique (512), qui présente une concentration plus élevée du marqueur que la première matière brute plastique (506), à la première matière brute plastique, lorsque la concentration du marqueur est inférieure à une concentration cible, en fonction de la concentration déterminée du marqueur dans la première matière brute plastique, de sorte qu'un mélange (514) de la première et de la deuxième matière brute plastique soit obtenu, présentant une concentration cible du marqueur ; et

une troisième unité d'alimentation (516) pour fournir une troisième matière brute plastique (518), qui ne contient pas le marqueur, au mélange (514) dans un rapport prédéterminé au mélange, de sorte que le matériau plastique (502) soit obtenu, contenant une proportion prédéterminée du mélange (514).

10. Système selon la revendication 9, comprenant en outre :

une deuxième unité de détection (520) pour déterminer une concentration du marqueur dans la deuxième matière brute plastique (512) avant de la fournir et/ou dans le mélange (514),
dans lequel la deuxième unité d'alimentation (510) est configurée pour ajuster la quantité de la deuxième matière brute plastique fournie (512) en fonction de la concentration déterminée du marqueur dans la deuxième matière brute plastique et/ou le mélange (514), de sorte que le mélange présente la concentration cible du marqueur.

FIG 1

FIG 2

FIG 3

# FIG 4

FIG 5

454

452

20

456

450

FIG 6

NIR-
LED-
PULS

NIR-
SIGNAL

INT 1
TAU 1

INT 1
TAU 1

INT 2
TAU 2

INT 2
TAU 2

t

EP 4 139 662 B1

FIG 7

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20180088103 A1
- US 20040196462 A1
- US 20200122360 A1

**In der Beschreibung aufgeführte Nicht-Patentliteratur**

- **BRUNNER S et al.** Automated sorting of polymer flakes: Fluorescence labeling and development of a measurement system prototype. *WASTE MANAGEMENT,* vol. 38, ISSN 0956-053X, 49-60